# EUROPEAN PATENT SPECIFICATION

(11) **EP 3 978 899 B1**
(45) Date of publication and mention of the grant of the patent: **22.11.2023**
(21) Application number: 20818808.6
(22) Date of filing: 22.05.2020
(51) Int. Cl.: G01N 5/02, G01N 1/00

(54) **SUBSTANCE DETECTION SYSTEM AND SUBSTANCE DETECTION METHOD**
SYSTEM ZUM NACHWEIS VON SUBSTANZEN UND VERFAHREN ZUM NACHWEIS VON SUBSTANZEN
SYSTÈME ET PROCÉDÉ DE DÉTECTION DE SUBSTANCE

(30) Priority: 03.06.2019 JP 2019103515
(43) Date of publication of application: 06.04.2022
(73) Proprietor: I-PEX Inc., Fushimi-ku, Kyoto-shi Kyoto 612-8024 (JP)
(72) Inventor: OGATA, Kenji, Fukuoka 838-0106 (JP); MATSUOKA, Keita, Fukuoka 838-0106 (JP)
(74) Representative: SSM Sandmair
(86) International application number: PCT/JP2020/020396
(87) International publication number: WO 2020/246276

(56) References cited:
- WO-A1-2019/123864
- WO-A2-2005/031300
- JP-A- H01 244 335
- JP-A- 2001 507 460
- JP-A- 2005 147 793
- JP-A- 2011 203 007
- US-A- 5 616 827
- US-A1- 2003 172 717
- US-A1- 2004 210 099
- US-A1- 2007 068 493
- US-A1- 2010 061 893
- US-A1- 2014 031 263
- US-A1- 2014 305 191
- US-A1- 2018 299 410

## Description

### Technical Field

The present disclosure relates to a substance detection system and a substance detection method.

### Background Art

Patent Literature 1 discloses an odor sensor that detects an odor substance attached to a substance attachment element by a change in the weight of the substance attachment element. This type of odor sensor has a limit to the amount of odor substance that can be attached to the substance attachment element. When the amount of attachment of the odor substance reaches the limit, the substance attachment element is incapable of having any more amount of odor substance attached thereto, and hence the substance attachment element is in a saturated state, lowering the accuracy of detection of the odor substance. Therefore, the odor sensor is required to once detach the odor substance from the substance attachment element.

For example, the odor sensor described in Patent Literature 1 introduces a sample gas into a chamber by executing a sequence of operations in a cycle in which the sample gas is introduced into the chamber and after leaving the chamber in this state for 300 seconds, air is introduced into the chamber for 300 seconds. By introducing air for 300 seconds, the sample gas within the chamber is expelled out for refreshment of the chamber (paragraph 0170). Other examples of prior art documents are US2004/210099, US2014/305191, US2014/031263, WO2005/031300 and US2007/068493.

### Citation List

### Patent Literature

Patent Literature 1: Japanese Patent No. 6508689

### Summary of Invention

### Technical Problem

In the above-described odor sensor, the sample gas within the chamber is required to be expelled out by air so as to detach the odor substance from the substance attachment element. However, in an environment in which the sample gas as a target of measurement cannot be expelled out (for example, an environment in which the sample gas is incessantly generated), there is an inconvenience that the detachment of the odor substance is insufficient, resulting in lowered detection accuracy of the odor substance.

The present disclosure has been made under such circumstances, and an objective thereof is to provide a substance detection system and a substance detection method that are capable of suppressing lowering of the detection accuracy of a target substance.

### Solution to Problem

To attain the object, a substance detection system according to a first aspect of the present disclosure is a substance detection system as defined in claim 1, and its dependent claims.

A substance detection method according to a second aspect of the present disclosure is defined in method claim 8.

### Advantageous Effects of Invention

According to the present disclosure, the gas supplier supplies the gas in the second space which is different in the concentration of the target substance from the first space, to the first space in which a substance sensor is disposed, whereby the concentration of the target substance around a substance sensor that detects the target substance is adjusted such that the concentration of the target substance around the substance sensor is increased when detecting the target substance, and is reduced when detaching the target substance. This makes it possible to prevent the amount of attachment of the target substance to the substance sensor from being saturated, and hence it is possible to suppress lowering of the accuracy of detection of the target substance.

### Brief Description of Drawings

FIG. 1 is a schematic view of the configuration of a substance detection system according to Embodiment 1 of the present disclosure;
FIG. 2 is a view of the configuration of a header of a substance sensor;
FIG. 3 is a block diagram of a control system of the substance detection system in FIG. 1;
FIG. 4 is a flowchart showing a substance detection method according to Embodiment 1 of the present disclosure;
FIG. 5 is a schematic view illustrating Operation 1 of the substance detection system according to Embodiment 1 of the present disclosure;
FIG. 6 is a schematic view illustrating Operation 2 of the substance detection system according to Embodiment 1 of the present disclosure;
FIG. 7A is a first timing diagram showing an operation of the substance detection system according to Embodiment 1 of the present disclosure;
FIG. 7B is a second timing diagram showing an operation of the substance detection system according to Embodiment 1 of the present disclosure;
FIG. 8 is a schematic view of the configuration of a substance detection system in a case where there are a plurality of second spaces;
FIG. 9 is a schematic view illustrating Operation 1 of a substance detection system according to Embodiment 2 of the present disclosure; and
FIG. 10 is a schematic view illustrating Operation 2 of the substance detection system according to Embodiment 2 of the present disclosure.

### Description of Embodiments

Hereafter, embodiments of the present disclosure are described in detail with reference to accompanying drawings. In each drawing, the same or equivalent element is denoted by the same reference numeral. Note that in the embodiments, "space" refers to a cubic region that is filled with a gas in which an odor is wafting, for example, the inside of a container, a room, or the like.

### Embodiment 1

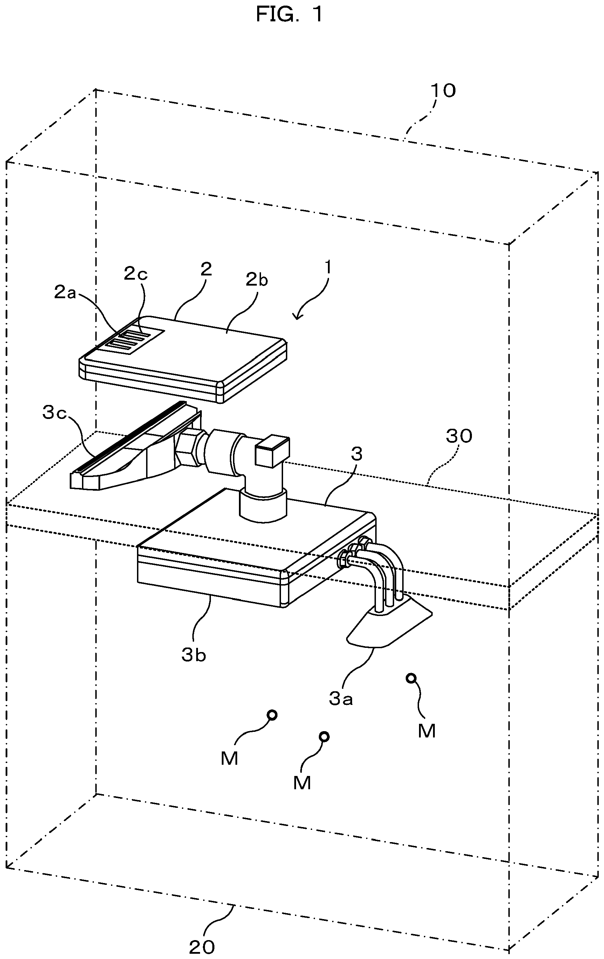

Referring to FIG. 1, a substance detection system 1 according to the present embodiment includes a substance sensor 2 that detects a target substance M contained in air and a pump 3 as a gas supplier. The target substance M includes, for example, an odor substance which is the source of an odor.

The substance detection system 1 according to the present embodiment requires two spaces, a first space 10 and a second space 20. The first space 10 and the second space 20 are partitioned by a wall 30. The substance sensor 2 is set in the first space 10. The pump 3 supplies a gas existing in the second space 20 to the first space 10.

Note that, in FIG. 1, for illustrative purposes, the first space 10 and the second space 20 are each illustrated as a rectangle of which sides are indicated by long and short dashed lines. However, in actuality, the first space 10 and the second space 20 may be spaces having larger expansions than the illustrated ones.

The first space 10 is, for example, a garret of a house, and the second space 20 is, for example, a living space of the house. When a comparison is made between the first space 10 and the second space 2, the first space 10 is higher in gas permeability to an outside than the second space 20, and the second space 20 is higher in hermeticity than the first space 10. Therefore, even when a substance as a detection target is fed to the first space 10, the substance is easy to be discharged or dispersed out of the first space 10, whereas the second space 20 is easy for the target substance M to remain therein.

In the present embodiment, the second space 20 is a target where substance detection is performed, and there is a possibility that the target substance M exists in the second space 20. The target substance M is fed from the second space 20 to the first space 10 for detection, and hence in a case where the target substance M exists in the second space 20, it is required that the concentration of the target substance M in the first space 10 is lower than that in the second space 20 (is desirably 0%).

Note that in a case where the target substance M does not exist in the first space 10 and the second space 20, the concentration of the target substance M in the first space 10 may be 0% which is equal to that in the second space 20. In other words, it is only required that the concentration of the target substance M is different between the first space 10 and the second space 20 in a case where the target substance M exists in at least one of the first space 10 or the second space 20.

The substance detection system 1 according to the present embodiment detects the target substance M contained in the gas in the second space 20, using the substance sensor 2 disposed in the first space 10. To detect the target substance M, the pump 3 feeds the gas existing in the second space 20 from the second space 20 to the first space 10.

The configuration of the substance detection system 1 is described in further detail. The substance sensor 2 includes a header 2a and a sensor body 2b. The header 2a has through holes 2c formed therethrough. The gas can pass through the through hole 2c.

The header 2a has an element having a sensitivity to the target substance M. The sensor body 2b includes a signal circuit that is capable of detecting information indicative of whether the target substance M has been attached, from the header 2a, and outputting the information as a signal, and a control circuit that controls the element of the header 2a.

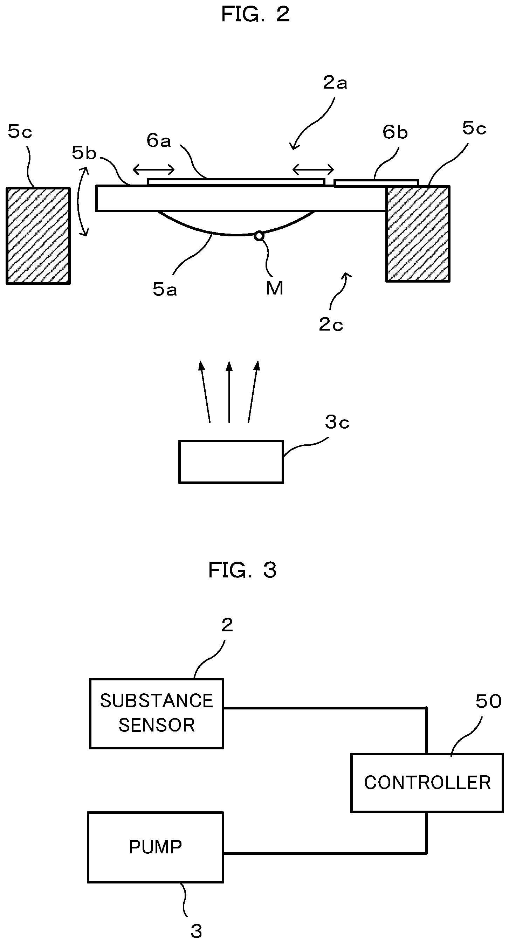

Referring to FIG. 2, the header 2a includes a substance attachment element 5a as the element mentioned above, a vibrator 5b, and a header body 5c. The target substance M contained in air is attached to the substance attachment element 5a. The vibrator 5b is a plate shaped member in the form of a cantilever beam. A driving piezoelectric element 6a capable of extending and contracting in a surface direction according to a voltage applied thereto is formed on one side of the vibrator 5b. The extension and contraction of the driving piezoelectric element 6a causes vibration of the vibrator 5b. Further, a detecting piezoelectric element 6b for detecting a vibration displacement is also formed on the one side of the vibrator 5b.

The substance attachment element 5a is formed on the vibrator 5b. When the target substance M is attached to the substance attachment element 5a, the weight of the substance attachment element 5a changes, and as a result, the total weight of the vibrator 5b changes. This change in the weight of the vibrator 5b changes the frequency characteristic of the vibrator 5b. The substance sensor 2 monitors for a change in the frequency characteristic of the vibrator 5b to thereby detect the target substance M. A change in the frequency characteristic of the vibrator 5b is observed through a change in a voltage signal output from the detecting piezoelectric element 6b (vibration frequency (for example, resonance frequency or natural frequency) of the vibrator 5).

Referring again to FIG. 1, in the present embodiment, the pump 3 includes a suction port 3a, a pump body 3b, and a blow-out port 3c. The suction port 3a is disposed in the second space 20, and the pump body 3b feeds a gas suctioned from the suction port 3a to the blow-out port 3c, by action of pressure. The blow-out port 3c is arranged such that it blows the gas directly against the header 2a of the substance sensor 2. More specifically, the blow-out port 3c is disposed in a state opposed to the substance attachment element 5a such that the gas blown out from the blow-out port 3c directly flows against the substance attachment element 5a.

Further, as described above, the substance sensor 2 detects existence of the target substance M from a change in the frequency characteristic of the vibrator 5b due to attachment of the target substance M to the substance attachment element 5a. However, the amount of attachment of the target substance M to the substance attachment element 5a has a limit and is saturated at a certain amount. When the substance attachment element 5a is in the saturated state, the target substance M is no longer attached to the substance attachment element 5a, which makes it impossible to measure the target substance M.

To overcome this problem, after detection of the target substance M, the target substance M attached to the substance attachment element 5a is detached therefrom for a predetermined time period. The detachment of the target substance M from the substance attachment element 5a is performed by causing the vibrator 5b to vibrate while stopping the blow-out of the gas against the substance attachment element 5a. The detachment of the target substance M from the substance attachment element 5a causes a change in the frequency characteristic of the vibrator 5b, which also makes it possible to detect removal of the target substance M from the substance attachment element 5a.

Referring to FIG. 3, the substance detection system 1 includes a controller 50. The controller 50 controls the substance sensor 2 and the pump 3. The controller 50 applies the voltage to the driving piezoelectric element 6a of the substance sensor 2 to thereby vibrate the vibrator 5b. The substance sensor 2 detects a vibration frequency of the vibrator 5b based on an output from the detecting piezoelectric element 6b. The substance sensor 2 outputs a signal indicative of the detection to the controller 50. The controller 50 detects a change in the frequency characteristic of the vibrator 5b based on the output from the substance sensor 2.

Further, the controller 50 performs on-off control of the pump 3. Specifically, the controller 50 turns on the pump 3 to feed the gas from the second space 20 to the first space 10. The controller 50 performs detection of the target substance M in the on-state of the pump 3. In the following description, the operation of vibrating the vibrator 5b while holding the pump 3 in the on-state is also referred to as the operation for detecting the target substance M.

When the controller 50 turns off the pump 3, the supply of the gas from the second space 20 to the first space 10 is stopped. The stoppage of the supply of the gas causes lowering of the concentration of the target substance M around the substance attachment element 5a. At this time, the target substance M is detached from the substance attachment element 5a. In the following, the operation of holding the pump 3 in an off-state is also referred to as the operation for detaching the target substance M.

As described above, the controller 50 turns on and off the pump 3 to thereby perform intermittent control in which the operation for detecting the target substance M and the operation for detaching the target substance M are repeated.

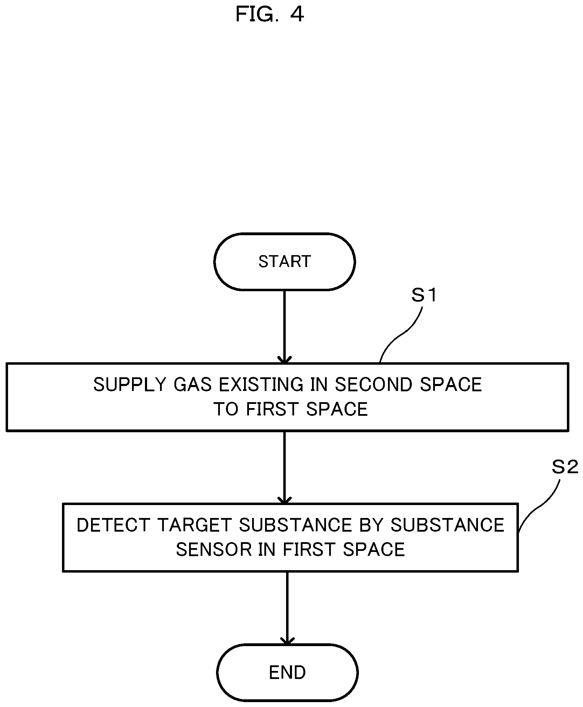

Next, an operation of the substance detection system 1 according to the embodiment of the present disclosure is described. In the method of this operation, first, as shown in FIG. 4, in a case where the target substance exists in at least one of the first space 10 or the second space 20, the gas existing in the second space 20 which is different in the concentration of the target substance M from the first space 10 is supplied to the first space 10 (step S1; first step). Then, the target substance M is detected by the substance sensor 2 disposed in the first space 10 (step S2; second step). With this, detection of the target substance M contained in the second space 20 is performed.

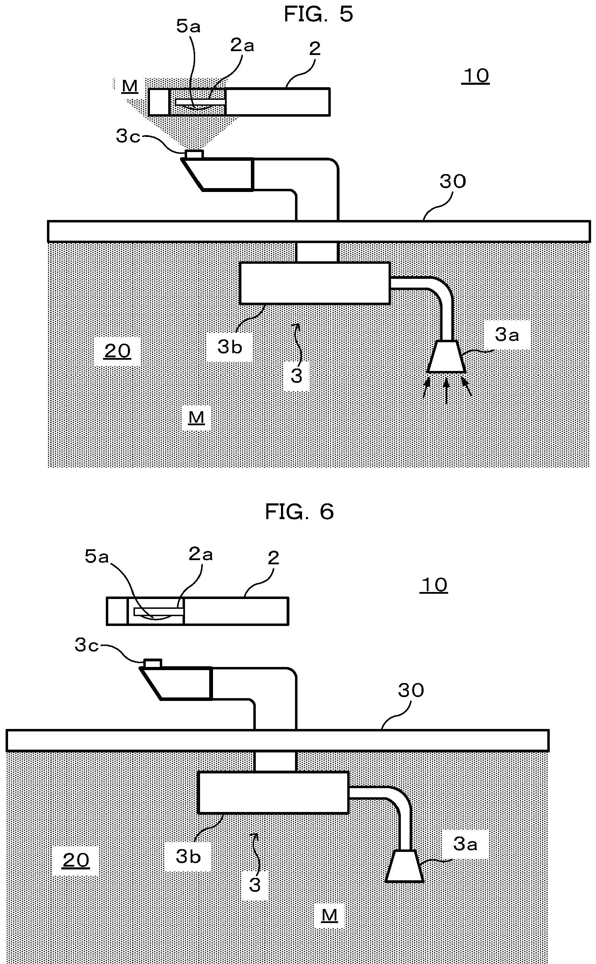

Further, as shown in FIG. 5, in the operation for detecting the target substance M, the controller 50 turns on the pump 3. This causes the pump 3 to operate to thereby supply the gas existing in the second space 20 to the first space 10. With this, the gas containing the target substance M is blown out from the blow-out port 3c of the pump 3, thereby attaching the target substance M to the substance attachment element 5a of the substance sensor 2. When the target substance M is attached, the frequency characteristic of the vibrator 5b changes. The controller 50 applies a voltage to the driving piezoelectric element 6a to cause the vibrator 5b to vibrate and monitor the vibration frequency of the vibrator 5b. The substance sensor 2 detects the target substance M from a change in the vibration frequency.

Further, as shown in FIG. 6, in the operation for detaching the target substance M, the controller 50 turns off the pump 3. This stops the supply of the gas existing in the second space 20 to the first space 10 to stop the gas containing the target substance M from being blow out from the blow-out port 3c of the pump 3, whereby the target substance M ceases to exist around the substance attachment element 5a of the substance sensor 2. As a result, the target substance M is detached from the substance attachment element 5a, whereby the substance attachment element 5a returns to an initial state in which the target substance M is not attached thereto. The controller 50 applies the voltage to the driving piezoelectric element 6a to thereby cause vibration of the vibrator 5b to promote detachment of the target substance M from the substance attachment element 5a.

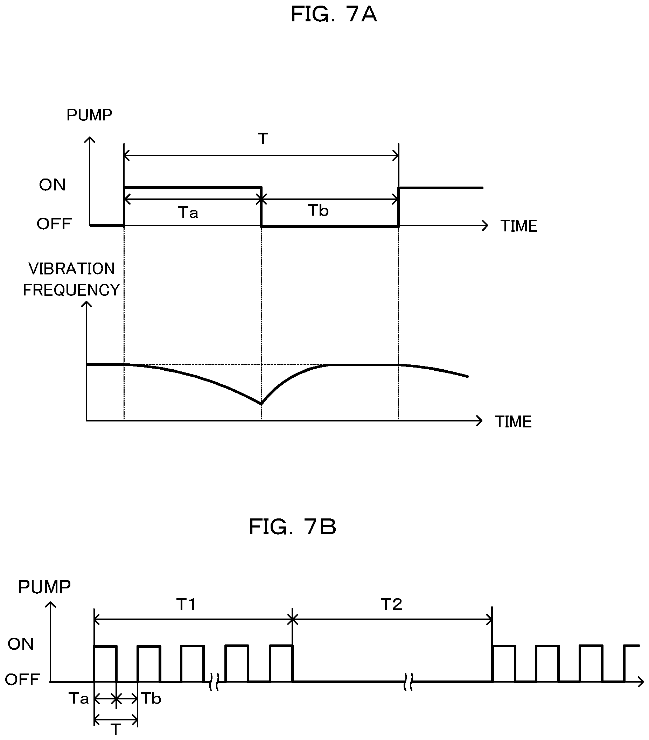

As shown in FIG. 7A, the controller 50 repeats a process in which the pump 3 is held in the on-state for a time period Ta and thereafter the pump 3 is held in the off-state, for a time period Tb, at a repetition period T. The time periods Ta and Tb can be set to a range of two to three seconds, for example.

The time period Ta is a time period during which the operation for detecting the target substance M is performed. During the time period Ta, the controller 50 turns on the pump 3 and also causes the vibrator 5b to vibrate, so as to detect a change in the vibration frequency of the vibrator 5b by monitoring an output from the detecting piezoelectric element 6b while causing the vibrator 5b to vibrate by application of the voltage to the driving piezoelectric element 6a. During the time period Ta, the target substance M is attached to the substance attachment element 5a to progressively lower the vibration frequency of the vibrator 5b. From this change in the vibration frequency, existence of the target substance M is detected.

Then, the following time period Tb is a time period during which the operation for detaching the target substance M is performed. During the time period Tb, the controller 50 turns off the pump 3. Then, the concentration of the target substance M around the substance attachment element 5a lowers to cause the target substance M to be detached from the substance attachment element 5a. At this time as well, the controller 50 applies the voltage to the driving piezoelectric element 6a to cause vibration of the vibrator 5b. This vibration makes it even easier for the target substance M to be detached from the substance attachment element 5a. In a case where the output of the voltage signal from the detecting piezoelectric element 6b is monitored in this state, as shown in FIG. 7A, the vibration frequency of the vibrator 5b increases due to detachment of the target substance M from the substance attachment element 5a.

Note that in the present embodiment, the description is given of a case where, as the target substance M is attached to the substance attachment element 5a, the vibration frequency of the vibrator 5b lowers whereas as the target substance M is detached from the substance attachment element 5a, the vibration frequency of the vibrator 5b increases, but the present disclosure is not limited to this. Whether or not the target substance M is attached to the substance attachment element 5a can be determined by an amplitude level of the vibrator 5b. For example, insofar as the substance sensor 2 is configured such that in a case where the target substance M is attached to the substance attachment element 5a, the amplitude level of the vibrator 5b lowers, whereas in a case where the target substance M is detached from the substance attachment element 5a, the amplitude level of the vibrator 5b increases, it is possible to determine whether or not the target substance M has been attached, by a difference in the amplitude level.

The controller 50 repeats the operation for detecting the target substance M and the operation for detaching the target substance M, as described above. With this, the substance detection system 1 is capable of performing the operation for detecting the target substance M for a long term, without saturating the amount of attachment of the target substance M to the substance attachment element 5a.

Further, in the present embodiment, in a case where the target substance M is difficult to be discharged from the first space 10, a discharge pump may be additionally provide as discharge means for discharging the gas from the first space 10.

Note that in the present embodiment, the first space 10 is assumed to be a garret of a housing and the second space 20 is assumed to be a living space of the housing, the present disclosure is not limited to this. For example, the first space 10 may be set to a space under eaves of a housing. The first space 10 and the second space 20 may be spaces formed in a building, or may be spaces formed in a facility or in an apparatus.

Further, the substance detection system 1 according to the present embodiment is capable of performing the operation for detecting the target substance M at intervals. As shown in FIG. 7B, it is possible to repeatedly execute the operation for detecting the target substance M for a time period Ta and the operation for detaching the target substance M for a time period Tb, during the time period T1, and stop the operation for detecting the target substance M and the operation for detaching the target substance M, during the following time period T2. By thus causing the substance detection system 1 to operate, even when the target substance M attached to the substance attachment element 5a remains in the time period T1, it is possible to create a time period to spare for detaching the target substance M by the time period T2. During the time period T2, to make the target substance M even easier to be detached from the substance attachment element 5a, the controller 50 may apply the voltage to the driving piezoelectric element 6a to cause vibration of the vibrator 5b.

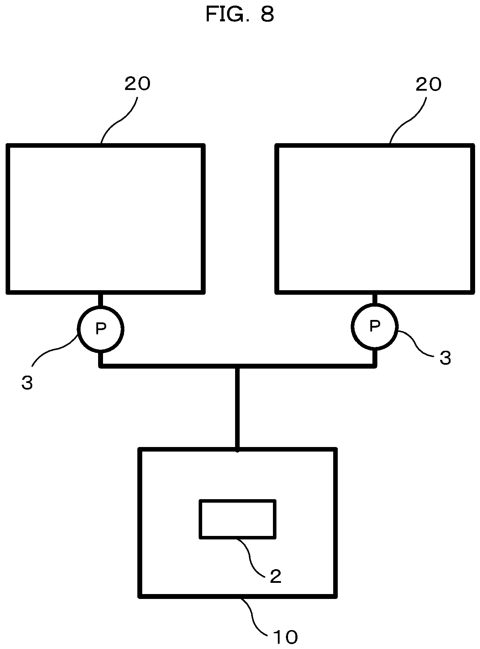

As described above, in the present embodiment, it is possible to provide a time period T2 to spare during which detection of the target substance M contained in the second space 20 is not executed. In this case, as shown in FIG. 8, a plurality of second spaces 20 may be provided, with the pump 3 being provided for each of the second spaces 20, whereby detection of the target substance M may be executed sequentially for the plurality of second spaces 20. In other words, as shown in FIG. 7B, in a case where detection of the target substance M is not executed in one of the second spaces 20 during the time period T2, the pump 3 for supplying the gas to the first space 10 may be switched so as to perform detection of the target substance M in the other of the second spaces 20 during the time period T2. Such a control can be applied even when there are three or more second spaces 20.

Note that, as the time periods Ta and Tb, it is required to set a plenty of time periods long enough to detect and detach the target substance M. Further, in the substance detection system 1 according to the present embodiment, the time period T2 during which the detection is not executed is not necessarily required. In particular, for a substance detection system 1 for detecting a target substance M which is very hazardous, it is desirable that detection of the target substance M is constantly executed.

### Embodiment 2

Next, Embodiment 2 of the present disclosure is described. A substance detection system 1 according to the present embodiment is configured to detect the target substance M that exists not in the second space 20 but in the first space 10.

The substance detection system 1 according to the present embodiment is the same as that according to Embodiment 1 in that it includes the substance sensor 2 and the pump 3 as a gas supplier. The substance sensor 2 is disposed in the first space 10. The pump 3 supplies a gas existing in the second space 20 to the first space 10. Details of the configurations of the substance sensor 2 and the pump 3 are the same as those in Embodiment 1 described above. Further, Embodiment 2 is also the same as Embodiment 1 in that the controller 50 controls the substance sensor 2 and the pump 3.

In the present embodiment, the first space 10 is a target where substance detection is performed, and there is a possibility that the target substance M exists in the first space 10. The gas in the second space 20 is fed to the first space 10 so as to remove the target substance M attached to the substance attachment element 5a, and hence, in a case where the target substance M exists in the first space 10, it is required that the concentration of the target substance M in the second space 20 is lower than that in the first space 10 (is desirably 0%).

Note that in the present embodiment as well, in a case where the target substance M does not exist in the first space 10 and the second space 20, the concentration of the target substance M in the first space 10 may be 0% which is equal to that in the second space 20. In other words, it is only required that the concentration of the target substance M is different between the first space 10 and the second space 20 in a case where the target substance M exists in at least one of the first space 10 or the second space 20.

The substance detection system 1 according to the present embodiment detects the target substance M contained in the gas in the first space 10, by using the substance sensor 2 disposed in the first space 10. In other words, the first space 10 is a space where measurement is to be performed, and in this point, the present embodiment is opposite to the embodiment described above in which the second space 20 is a target where substance measurement is to be performed.

In the present embodiment as well, the controller 50 applies a voltage to the driving piezoelectric element 6a to thereby vibrate the vibrator 5b, and detect the vibration frequency of the vibrator 5b based on an output from the detecting piezoelectric element 6a. The controller 50 detects a change in the frequency characteristic of the vibrator 5b based on the output.

Further, in the present embodiment as well, the controller 50 performs on-off control of the pump 3. Specifically, the controller 50 performs detection of the target substance M in the off-state of the pump 3. In the following description, the operation of vibrating the vibrator 5b while holding the pump 3 in the off-state is also referred to as the operation for detecting the target substance M.

When the controller 50 turns on the pump 3, a gas is supplied from the second space 20 to the first space 10. The gas contains a lower concentration of the target substance M than in the first space 10, and hence after the the supply of the gas is started, the concentration of the target substance M around the substance attachment element 5a is lowered. At this time, the target substance M is detached from the substance attachment element 5a. In the following, the operation of vibrating the vibrator 5b while holding the pump 3 in the on-state is also referred to as the operation for detaching the target substance M.

As described above, the controller 50 performs intermittent control in which the operation for detecting the target substance M and the operation for detaching the target substance M are repeated.

Next, an operation of the substance detection system 1 according to the embodiment of the present disclosure is described.

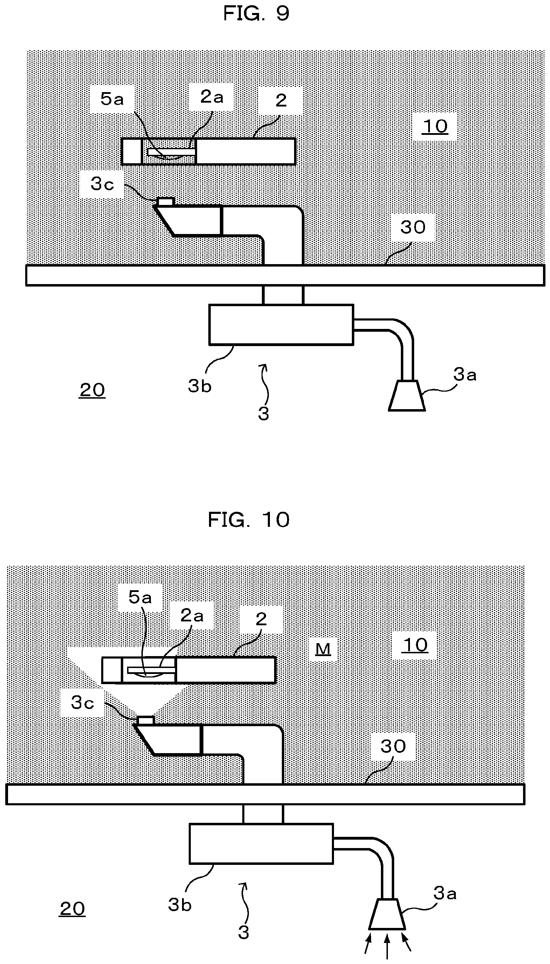

More specifically, as shown in FIG. 9, in the operation for detecting the target substance M, the pump 3 is stopped to stop the supply of the gas existing in the second space 20 to the first space 10, so that the blow-out of the gas from the blow-out port 3c of the pump 3 is stopped. In this state, the target substance M is attached to the substance attachment element 5a of the substance sensor 2 existing in the first space 10. When the target substance M is attached, the frequency characteristic of the vibrator 5b changes. The controller 50 applies the voltage to the driving piezoelectric element 6a to cause vibration of the vibrator 5b, and monitors the vibration frequency of the vibrator 5b using the detecting piezoelectric element 6b. The substance sensor 2 detects the target substance M from a change in the vibration frequency.

As shown in FIG. 10, in the operation for detaching the target substance M, the pump 3 operates to supply the gas existing in the second space 20 to the first space 10. This causes the gas containing a lower concentration of the target substance M to be blown out from the blow-out port 3c of the pump 3, whereby the concentration of the target substance M around the substance attachment element 5a of the substance sensor 2 is lowered. As a result, the target substance M is detached from the substance attachment element 5a. The controller 50 applies the voltage to the driving piezoelectric element 6a to thereby cause vibration of the vibrator 5b to promote detachment of the target substance M from the substance attachment element 5a.

The controller 50 thus repeats the operation for detecting the target substance M and the operation for detaching the target substance M. With this, the substance detection system 1 according to the present embodiment as well is capable of continuously performing the operation for detecting the target substance M without saturating the amount of attachment of the target substance M to the substance attachment element 5a.

Note that in the present embodiment, it is only required that the gas is supplied from the second space 20 to around the substance sensor 2 for a certain time period. As the time period over which the gas is supplied from the second space 20, a time period is secured which is long enough to cause the target substance M to be detached from the substance attachment element 5a.

As described in detail heretofore, according to the embodiments, by causing the pump 3 to supply the gas in the second space 20 which is different in the concentration of the target substance M from the first space 10 to the first space 10 in which the substance sensor 2 is disposed, the concentration of the target substance M around the substance sensor 2 for detecting the target substance M is adjusted such that it is made higher when detecting the target substance M and lower when detaching the target substance M. This makes it possible to prevent the amount of attachment of the target substance M in the substance sensor 2 from being saturated, and hence it is possible to suppress lowering of the accuracy of detection of the target substance M.

In the substance detection systems 1 according to the embodiments described above, by turning on or off the pump 3 and allowing time to elapse, the substance attached to the substance attachment element 5a can be caused to be detached, and hence, for example, it is possible to dispense with such a special mechanism as will supply a nitrogen gas to the substance attachment element 5a to cause the target substance M to be detached therefrom.

Further, according to the embodiment described above, the pump 3 is configured to intermittently supply a gas existing in the second space 20 to the substance attachment element 5a. With this configuration, it is possible to prevent the state of attachment of the target substance M being saturated due to constant exposure of the substance attachment element 5a to the target substance M.

Note that the intermittent control is executed for a certain time period, but this is not limitative. By monitoring a detection result, and when the vibration frequency of the vibrator 5b has converged to a predetermined range, the operation for detection may be stopped and the operation for detaching the target substance M may be executed.

Further, according to the embodiments, the substance attachment element 5a of the substance sensor 2 is disposed on a flow path of the gas blown out from the blow-out port 3c of the pump 3. With this configuration, it is possible to increase the amount of attachment of the target substance M to the substance attachment element 5a, and hence it is possible to enhance the accuracy of detection of the target substance M.

Further, according to the embodiments, by causing vibration of the vibrator 5b, the target substance M attached to the substance attachment element 5a is detached. With this operation, it is possible to detach the target substance M from the substance attachment element 5a positively and in a short time period.

Further, in the substance detection systems 1 according to the embodiments, the vibrator 5b in the form of a cantilever beam is vibrated, and the frequency characteristic of the vibrator 5b is detected. However, the present disclosure is not limited to this. For example, a quarts resonator may be vibrated. Further, the substance sensor 2 is not limited to one having the vibrator 5b. The substance sensor 2 can be applied insofar as it detects the target substance M.

Note that in the above embodiments, the vibrator 5b is configured to be a plate shaped member in the form of a cantilever beam, the present disclosure is not limited to this. The vibrator 5b may be a plate shaped member in the form of a double-sided beam, or may be a member in the form of a beam having three or more supported ends. Further, the driving piezoelectric element 6a and the detecting piezoelectric element 6b may be provided not at one end but at both ends of a beam.

Further, in the embodiments described above, the gas supplier is the pump 3. By using the pump 3, it is possible to quickly perform and stop the supply of the gas to the substance attachment element 5a. However, the present disclosure is not limited to this. The gas supplier may be a blower fan that blows a gas from the second space 20 to the first space 10.

The target substance M according to the embodiments includes a wide variety of substances. For example, the target substance M includes a life odor in a living space, a pet odor, a perfume, a garbage odor, a burning odor, and so forth. The operation of the substance detection system 1 can be properly adjusted according to the type of the target substance M as the detection target or the purpose of detection. The time periods Ta, Tb, T1, and T2 are typical examples of the adjustment.

Note that the substance detection systems 1 according to the embodiments are each configured to detect the target substance M and monitor for generation of the target substance M. However, the present disclosure is not limited to this. For example, an apparatus may be controlled by using a results of detection. For example, when the target substance M is detected, a ventilation fan may be turned to ventilate the second space 20, or occurrence of a fire may be notified by detecting a target substance M generated when an object is burnt.

Note that in the embodiments, the first space 10 and the second space 20 are partitioned by the wall 30, but the present disclosure is not limited to this. Insofar as the first space 10 and the second space 20 are sufficiently apart from each other, or there is no fear of mixing of gases existing in the respective spaces, the wall 30 can be dispensed with. Basically, it is only required that the concentration of the target substance M is different between the first space 10 and the second space 20. More desirably, it is only required that the target substance M exists in at least one of the first space 10 or the second space 20.

Further, in the above embodiments, only one type of substance is set as the target substance M, the present disclosure is not limited to this. There may be a plurality of types of target substances M. In this case, a substance sensor 2 may be provided for each of the target substances M. The substance detection system 1 may be configured to detect an odor formed by a plurality of kinds of target substances M.

This application claims the benefit of Japanese Patent Application No. 2019-103515, filed on June 3, 2019.

### Industrial Applicability

The present disclosure can be applied to detection of a target substance, which is selectively set from substances wafting in the air or various gases, such as an odor substance.

### Reference Signs List

- 1: Substance detection system
- 2: Substance sensor
- 2a: Header
- 2b: Sensor body
- 2c: Through hole
- 3: Pump (gas supplier)
- 3a: Suction port
- 3b: Pump body
- 3c: Blow-outport
- 5a: Substance attachment element
- 5b: Vibrator
- 5c: Header body
- 6a: Driving piezoelectric element
- 6b: Detecting piezoelectric element
- 10: First space
- 20: Second space
- 30: Wall
- 50: Controller
- M: Target substance

## Claims

1. A substance detection system (1) comprising:
A substance sensor (2) that is disposed in a first space (10) and detects a target substance (M);
a gas supplier (3) that supplies a gas in a second space (20) to the first space (10); and
a controller (50) configured to control the substance sensor (2) and the gas supplier (3),
wherein
the substance sensor (2) includes a substance attachment element (5a) to which the target substance (M) attaches,
the gas supplier (3) includes a blow-out port (3c) from which the gas existing in the second space (20) is blown out into the first space (10), and
the substance attachment element (5a) is disposed on a flow path of the gas blown out from the blow-out port (3c),
the substance sensor (2) includes a vibrator (5b) that has the substance attachment element (5a) mounted thereon and vibrates to detach the target substance (M) attached to the substance attachment element (5a),
**characterized in that**
the controller (50) is configured to repeat a process in which the gas supplier (3) is held in an on-state for a first predetermined period (Ta) and thereafter is held in an off-state for a second predetermined period (Tb), at a repetition period (T), wherein
the substance sensor (2) is configured to detect attachment of the target substance (M) to the substance attachment element (5a) for the first predetermined period (Ta), based on a change in a frequency characteristic of the vibrator, and
the vibrator (5b) is configured to vibrate for detaching the target substance (M) from the substance attachment element (5a) while the blow-out of the gas is stopped for the second predetermined period (Tb).

2. The substance detection system according to claim 1, wherein the gas supplier intermittently supplies the gas existing in the second space to the first space.

3. The substance detection system according to claim 2, wherein
the second space is a plurality of the second spaces,
the gas supplier is provided for each of the plurality of second spaces, and
the second spaces to which the gas is supplied to the first space are switchable among the plurality of second spaces.

4. The substance detection system according to claim 1, wherein the substance attachment element is provided such that the gas blown out from the blow-out port directly flows against the substance attachment element.

5. The substance detection system according to any one of claims 1 to 4, wherein the gas supplier is a pump.

6. The substance detection system according to any one of claims 1 to 5, wherein the first space and the second space are different in gas permeability to an outside.

7. The substance detection system according to any one of claims 1 to 6, wherein one of the first space or the second space is provided with discharge means for discharging the gas.

8. A substance detection method of detecting a target substance (M) using the substance detection system (1) according to claim 1, comprising:
a first step of supplying, by the gas supplier (3) of the substance detection system (1) according to claim 1, in a case where the target substance (M) exists in at least one of the first space (10) or the second space (20) of the substance detection system (1) according to claim 1, a gas existing in the second space (20) that is different in a concentration of the target substance (M) from the first space (10), to the first space (10); and
a second step of detecting the target substance (10) by the substance sensor (2) of the substance detection system (1) according to claim 1 disposed in the first space (10).

## Patentansprüche

1. Substanzerfassungssystem (1), umfassend:
einen Substanzsensor (2), der in einem ersten Raum (10) angeordnet ist und eine Zielsubstanz (M) erfasst;
einen Gaszuführer (3), der dem ersten Raum (10) ein Gas in einem zweiten Raum (20) zuführt, und
ein Steuergerät (50), das so konfiguriert ist, dass es den Substanzsensor (2) und den Gaszuführer (3) steuert,
wobei
der Substanzsensor (2) ein Substanzbefestigungselement (5a) enthält, an dem die Zielsubstanz (M) befestigt wird,
der Gaszuführer (3) eine Ausblasöffnung (3c) aufweist, aus der das im zweiten Raum (20) vorhandene Gas in den ersten Raum (10) ausgeblasen wird, und
das Substanzbefestigungselement (5a) auf einem Strömungsweg des aus der Ausblasöffnung (3c) ausgeblasenen Gases angeordnet ist,
der Substanzsensor (2) einen Vibrator (5b) enthält, an dem das Substanzbefestigungselement (5a) angebracht ist und der vibriert, um die an dem Substanzbefestigungselement (5a) befestigte Zielsubstanz (M) abzulösen,
**dadurch gekennzeichnet, dass**
das Steuergerät (50) so konfiguriert ist, dass es einen Prozess wiederholt, bei dem der Gaszuführer (3) für eine erste vorbestimmte Zeitspanne (Ta) in einem Ein-Zustand gehalten wird und danach für eine zweite vorbestimmte Zeitspanne (Tb) in einem Aus-Zustand gehalten wird, mit einer Wiederholungsperiode (T), wobei
der Substanzsensor (2) so konfiguriert ist, dass er die Befestigung der Zielsubstanz (M) an das Substanzbefestigungselement (5a) für die erste vorbestimmte Periode (Ta) auf der Grundlage einer Änderung einer Frequenzcharakteristik des Vibrators erfasst, und
der Vibrator (5b) so konfiguriert ist, dass er vibriert, um die Zielsubstanz (M) von dem Substanzbefestigungselement (5a) zu lösen, während das Ausblasen des Gases für die zweite vorbestimmte Periode (Tb) gestoppt wird.

2. Substanzerfassungssystem nach Anspruch 1, wobei der Gaszuführer das im zweiten Raum vorhandene Gas intermittierend dem ersten Raum zuführt.

3. Substanzerfassungssystem nach Anspruch 2, wobei
der zweite Raum eine Vielzahl von zweiten Räumen ist,
der Gaszuführer für jeden der Vielzahl von zweiten Räumen vorgesehen ist, und
die zweiten Räume, aus denen das Gas dem ersten Raum zugeführt wird, unter der Vielzahl der zweiten Räume umschaltbar sind.

4. Substanzerfassungssystem nach Anspruch 1, wobei das Substanzbefestigungselement so vorgesehen ist, dass das aus der Ausblasöffnung ausgeblasene Gas direkt gegen das Substanzbefestigungselement strömt.

5. Substanzerfassungssystem nach einem der Ansprüche 1 bis 4, wobei der Gaszuführer eine Pumpe ist.

6. Substanzerfassungssystem nach einem der Ansprüche 1 bis 5, wobei der erste Raum und der zweite Raum eine unterschiedliche Gasdurchlässigkeit nach außen aufweisen.

7. Substanzerfassungssystem nach einem der Ansprüche 1 bis 6, wobei einer aus dem ersten Raum oder dem zweiten Raum mit Auslassmitteln zum Auslassen des Gases vorgesehen ist.

8. Substanzerfassungsverfahren zum Erfassen einer Zielsubstanz (M) unter Verwendung des Substanzerfassungssystem (1) nach Anspruch 1, umfassend:
einen ersten Schritt des Zuführens eines in dem zweiten Raum (20) vorhandenen Gases, das sich in einer Konzentration der Zielsubstanz (M) von dem ersten Raum (10) unterscheidet, zu dem ersten Raum (10) durch den Gaszuführer (3) des Substanzerfassungssystem (1) nach Anspruch 1 in einem Fall, in dem die Zielsubstanz (M) in zumindest einem aus dem ersten Raum (10) und dem zweiten Raum (20) des Substanzerfassungssystems (1) nach Anspruch 1 vorhanden ist; und
einen zweiten Schritt des Erfassens der Zielsubstanz (10) durch den im ersten Raum (10) angeordneten Substanzsensor (2) des Substanzerfassungssystems (1) nach Anspruch 1.

## Revendications

1. Système de détection de substance (1), comprenant :
un capteur de substance (2) qui est disposé dans un premier espace (10) et détecte une substance cible (M) ;
un dispositif d'alimentation en gaz (3) qui effectue l'alimentation en un gaz, dans un second espace (20), au premier espace (10) ; et
une unité de commande (50) configurée pour commander le capteur de substance (2) et le dispositif d'alimentation en gaz (3), dans lequel
le capteur de substance (2) inclut un élément de fixation de substance (5a) auquel la substance cible (M) se fixe,
le dispositif d'alimentation en gaz (3) inclut un orifice de soufflage (3c) depuis lequel le gaz existant dans le second espace (20) est soufflé dans le premier espace (10), et
l'élément de fixation de substance (5a) est disposé sur un chemin d'écoulement du gaz soufflé depuis l'orifice de soufflage (3c),
le capteur de substance (2) inclut un vibrateur (5b) qui a l'élément de fixation de substance (5a) monté sur celui-ci et vibre pour séparer la substance cible (M) fixée à l'élément de fixation de substance (5a),
**caractérisé en ce que**
l'unité de commande (50) est configurée pour répéter un processus dans lequel le dispositif d'alimentation en gaz (3) est maintenu dans un état en marche pendant une première période prédéterminée (Ta) et après cela est maintenu dans un état en arrêt pendant une seconde période prédéterminée (Tb), à une période de répétition (T), dans lequel
le capteur de substance (2) est configuré pour détecter la fixation de la substance cible (M) à l'élément de fixation de substance (5a) pendant la première période prédéterminée (Ta), sur la base d'un changement d'une caractéristique de fréquence du vibrateur, et
le vibrateur (5b) est configuré pour vibrer pour séparer la substance cible (M), à partir de l'élément de fixation de substance (5a), alors que le soufflage du gaz est arrêté pendant la seconde période prédéterminée (Tb).

2. Système de détection de substance selon la revendication 1, dans lequel le dispositif d'alimentation en gaz effectue, de façon intermittente, l'alimentation en le gaz, existant dans le second espace, au premier espace.

3. Système de détection de substance selon la revendication 2, dans lequel
le second espace est une pluralité des seconds espaces,
le dispositif d'alimentation en gaz est prévu pour chacun de la pluralité de seconds espaces, et
les seconds espaces depuis lesquels l'alimentation en le gaz est effectuée au premier espace sont changeables parmi la pluralité de seconds espaces.

4. Système de détection de substance selon la revendication 1, dans lequel l'élément de fixation de substance est prévu de telle sorte que le gaz soufflé depuis l'orifice de soufflage s'écoule directement contre l'élément de fixation de substance.

5. Système de détection de substance selon l'une quelconque des revendications 1 à 4, dans lequel le dispositif d'alimentation en gaz est une pompe.

6. Système de détection de substance selon l'une quelconque des revendications 1 à 5, dans lequel le premier espace et le second espace sont différents en perméabilité aux gaz vers un extérieur.

7. Système de détection de substance selon l'une quelconque des revendications 1 à 6, dans lequel un du premier espace ou du second espace est pourvu de moyens d'évacuation pour évacuer le gaz.

8. Procédé de détection de substance, de la détection d'une substance cible (M) en utilisant le système de détection de substance (1) selon la revendication 1, comprenant :
une première étape, de l'alimentation, par le dispositif d'alimentation en gaz (3) du système de détection de substance (1) selon la revendication 1, dans un cas où la substance cible (M) existe dans au moins un du premier espace (10) ou du second espace (20) du système de détection de substance (1) selon la revendication 1, en un gaz existant dans le second espace (20), qui est différent en concentration de la substance cible (M) du premier espace (10), au premier espace (10) ; et
une seconde étape, de la détection de la substance cible (10), par le capteur de substance (2) du système de détection de substance (1) selon la revendication 1 disposé dans le premier espace (10).
